(19) **Europäisches Patentamt / European Patent Office / Office européen des brevets**

(11) **EP 1 166 747 B1**

(12) **FASCICULE DE BREVET EUROPEEN**

(45) Date de publication et mention de la délivrance du brevet:
**18.04.2007 Bulletin 2007/16**

(51) Int Cl.:
*A61K 8/02* (2006.01)  *A61K 8/04* (2006.01)
*A61Q 19/10* (2006.01)  *C11D 17/00* (2006.01)

(21) Numéro de dépôt: **01401390.8**

(22) Date de dépôt: **28.05.2001**

(54) **Crème cosmétique moussante**

Selbstschäumende kosmetische Creme

Foaming cosmetic cream

(84) Etats contractants désignés:
**AT BE CH CY DE DK ES FI FR GB GR IE IT LI LU MC NL PT SE TR**

(30) Priorité: **23.06.2000 FR 0008085**

(43) Date de publication de la demande:
**02.01.2002 Bulletin 2002/01**

(73) Titulaire: **L'ORÉAL**
**75008 Paris (FR)**

(72) Inventeurs:
• **Guillou, Véronique**
**92160 Antony (FR)**
• **Morancais, Jean-Luc**
**77330 Ozoir la Ferriere (FR)**

(74) Mandataire: **Rasson, Catherine**
**L'OREAL - D.I.P.I.**
**25-29 Quai Aulagnier**
**92600 Asnières (FR)**

(56) Documents cités:
**EP-A- 0 339 994**  **WO-A-99/51716**
**US-A- 5 320 783**  **US-A- 5 756 108**

• **C. RODRIGUEZ ET AL.: "Cubic-phase-based concentrated emulsions" JOURNAL OF COLLOID AND INTERFACE SCIENCE, no. 223, 2000, pages 197-204, XP000979610**

Remarques:
Le dossier contient des informations techniques présentées postérieurement au dépôt de la demande et ne figurant pas dans le présent fascicule.

**Description**

La présente invention a trait à une composition moussante rinçable constituant une crème pour application topique, contenant un système tensioactif particulier et présentant une bonne stabilité physique jusqu'à au moins 45°C, ainsi qu'à son utilisation dans les domaines cosmétique ou dermatologique, notamment comme produits de nettoyage ou de démaquillage de la peau, du cuir chevelu et/ou des cheveux.

Le nettoyage de la peau est très important pour le soin du visage. Il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

On connaît plusieurs grands types de produits de nettoyage de la peau : les lotions et gels aqueux détergents moussants, les huiles et gels anhydres nettoyants rinçables et les crèmes moussantes.

Les huiles et gels anhydres rinçables ont une action nettoyante grâce aux huiles contenues dans ces formulations. Ces huiles permettent la solubilisation des résidus gras et la dispersion des pigments de maquillage. Ces produits sont efficaces et bien tolérés. Ils présentent l'inconvénient d'être lourds, de ne pas mousser et de ne pas conférer de sensation de fraîcheur à l'application, ce qui est pénalisant d'un point de vue cosmétique.

Par ailleurs, les lotions et gels aqueux détergents moussants ont une action nettoyante grâce aux tensioactifs qui mettent en suspension les résidus gras et les pigments des produits de maquillage. Ils sont efficaces et agréables à utiliser du fait qu'ils moussent et qu'ils sont facilement éliminés. Toutefois, les lotions sont généralement assez fluides, ce qui rend leur manipulation parfois délicate, et il est difficile d'épaissir les gels tout en gardant de bonnes propriétés moussantes.

Pour obtenir de bonnes performances moussantes tout en ayant une composition épaisse, on a cherché à préparer des crèmes moussantes. Toutefois, les crèmes moussantes présentent l'inconvénient d'être souvent instables à chaud.

On entend ici par « crèmes moussantes » des compositions opaques, visqueuses, souvent commercialisées en tube et constituées généralement d'un milieu aqueux contenant un mélange de tensioactifs tels que les sels d'acides gras (savons) ou les tensioactifs synthétiques anioniques, non ioniques, amphotères, et d'autres additifs tels que par exemple des polymères, des polyols, des charges.

Ces crèmes destinées en particulier au nettoyage de la peau développent de la mousse quand elles sont mélangées avec de l'eau. Elles peuvent être utilisées de deux façons :

- la première utilisation consiste à étaler la crème dans les mains, à l'appliquer sur le visage ou sur le corps puis à la masser en présence d'eau pour développer la mousse directement sur le visage ou le corps.
- l'autre utilisation possible de ce type de produit consiste à développer la mousse dans les paumes des mains avant d'être appliquée sur le visage ou le corps.

Dans les deux cas, la mousse est ensuite rincée.

La plupart des crèmes moussantes actuellement disponibles dans le commerce sont instables au-dessus de 40°C. Cela signifie que, si elles sont entreposées quelques jours à cette température, elles présentent une démixtion macroscopique, aboutissant à une séparation en au moins deux phases. Les crèmes ainsi demixées à une température nettement supérieure à la température ambiante se révèlent hétérogènes après retour à la température ambiante et donc inutilisables du fait de la dégradation de la texture et des propriétés moussantes. On entend ici par température ambiante une température tempérée, c'est-à-dire d'environ 20 à 25°C.

Or, il est indispensable que ce type de produit soit stable sur une large plage de températures. En effet, au cours de sa vie, le produit peut être exposé à des températures allant de -20°C à +45°C minimum selon les conditions de climat, de stockage et/ou de transport. Par exemple, il faut qu'une crème transportée dans une voiture qui risque de rester longtemps sous le soleil, c'est-à-dire à une température atteignant facilement 50°C conserve sa stabilité. Il faut aussi que ces crèmes moussantes puissent être utilisées dans les pays chauds sans que leur transport et leur conservation ne posent de problème.

Il est bien connu qu'il est possible d'empêcher cette séparation de phases d'une crème moussante en augmentant, par addition de polymères ou de charges, la consistance du produit soumis à des températures de +40° à +45°C. Toutefois, dans ce cas, le produit devient très rigide à température ambiante tempérée et ne correspond plus aux propriétés recherchées pour l'application sur la peau, et notamment il devient difficile de le mélanger à l'eau et de le faire mousser.

Il subsiste donc le besoin d'une crème moussante, stable jusqu'à au moins 45°C, dont l'aspect de crème soit maintenu à température ambiante même après passage à une température plus élevée et ayant de bonnes caractéristiques de moussage.

La demanderesse a découvert de manière surprenante qu'on pouvait atteindre le but de l'invention et obtenir une composition moussante se présentant sous forme d'une crème et ayant une bonne stabilité, même à des températures de +40 - +45°C, en utilisant un système tensioactif tel qu'au moins une phase paracristalline de type hexagonale directe apparaisse quand ladite composition est chauffée à une température supérieure à 30°C et que cette phase paracristalline reste présente jusqu'à au moins 45°C.

Le fait qu'une phase paracristalline de type hexagonale directe apparaisse quand ladite composition est chauffée à une température supérieure à 30°C et que cette phase paracristalline reste présente jusqu'à au moins 45°C signifie que cette phase est présente à une température comprise entre 30°C et au moins 45°C.

Il n'est pas nécessaire que cette phase paracristalline soit présente à température ambiante mais elle doit impérativement apparaître au delà d'une température comprise entre 30°C et 45°C.

Les crèmes moussantes, qui ne présentent pas une organisation de phases, telle que citée ci-dessus, ne sont généralement pas stables à 45°C. A cette température, elles subissent une démixtion macroscopique entre au moins deux phases et elles sont ensuite inappropriées à l'utilisation recherchée quand elles sont de nouveau à la température ambiante.

Ainsi, la présente demande a pour objet une composition moussante constituant une crème pour application topique, contenant dans un milieu aqueux, un système tensioactif tel qu'au moins une phase paracristalline de type hexagonale directe apparaisse quand la température augmente au-delà de 30°C et que cette phase paracristalline reste présente jusqu'à au moins 45°C, telle que définie dans la revendication 1.

La composition obtenue constitue une crème opaque qui a de très bonnes propriétés cosmétiques (douceur, onctuosité), mousse bien et a une bonne stabilité pendant longtemps et à températures élevées.

La ou les phases paracristallines présentes au-delà de +30°C sont de type hexagonale directe ou un mélange de cette phase avec une phase de type lamellaire.

On donne dans la présente demande aux termes de phase lamellaire, phase hexagonale directe, les sens que leur donne habituellement l'homme du métier.

Ainsi, on entend par phase lamellaire (phase D selon EKWALL, voir Advances in Liquid Crystals, vol. 1, page 1-143, Acad. Press, 1975, Ed. G.H. Brown), une phase cristal liquide à symétrie plane, comprenant plusieurs bicouches d'amphiphile disposées en parallèle et séparées par un milieu liquide qui est généralement de l'eau.

On entend par phase hexagonale directe (phase F selon EKWALL, voir Advances in Liquid Crystals, vol. 1, page 1-143, Acad. Press, 1975, Ed. G.H. Brown), une phase cristal liquide correspondant à une arrangement hexagonal de cylindres parallèles constitués d'un amphiphile et séparés par un milieu liquide qui est généralement de l'eau. Dans une phase hexagonale directe, le milieu continu est aqueux.

Une description plus précise de ces phases peut être trouvée dans la Revue Française des Corps Gras, n°2, Février 1969, p 87 à 111, (Lachampt et Vila, « Textures des phases paracristallines »).

Pour identifier les phases constitutives de la crème, on peut utiliser différentes techniques, et notamment (1) les mesures par diffraction des rayons-X aux petits angles et aux grands angles et (2) l'observation par microscopie optique en lumière polarisée.

Technique de diffraction des rayons-X

La technique de diffraction des rayons-X est connue comme étant l'une des plus pertinentes pour mettre en évidence les organisations de phases paracristallines, en particulier au sein d'un échantillon. Les mesures par diffraction des rayons X peuvent être effectuées à l'aide d'un générateur CGR Sigma 2060 équipé d'un tube Inel à anticathode Cu et d'une chambre à focalisation linéaire montée en transmission symétrique. Les échantillons sont introduits à température ambiante dans une cellule de mesure obturée par des fenêtres en Mylar ou en Capton et placée dans un porte-échantillon thermorégulé.

Les spectres de diffraction obtenus avec une longueur d'onde $\lambda$ = 1,54 Angströms (Raie $K\alpha$ du cuivre) sont enregistrés à l'aide d'un écran photostimulable au phosphore scanné par un module de balayage laser Molecular Dynamics PhosphorImager PSI. La distance détecteur / échantillon est réglée à 133 mm ce qui donne accès à des distances réticulaires comprises entre environ 3 et 110 Angströms. Les spectres sont enregistrés à différentes températures fixes.

Avec cette technique, les phases paracristallines sont caractérisées par la présence, aux petits angles de diffraction, d'une série de plusieurs raies fines dues à des réflections de Bragg et correspondant à des distances : d1, d2.....dn avec des rapports de distance d1/d1, d1/d2, ....., d1/dn qui sont caractéristiques de chaque type de phase, comme indiqué par exemple dans « La structure des colloïdes d'association, I. Les phases liquides cristallines des systèmes amphiphile-eau », V. Luzzati, H. Mustachi, A. Skoulios et F. Husson, Acta Cryst. (1960), 13, 660-667 ou dans Biochimica et Biophysica Acta (1990), 1031, p. 1 à 69, de J.M. Seddon. Ainsi, pour une phase de structure lamellaire et en particulier pour la phase paracristalline de type lamellaire fluide généralement désignée par $L\alpha$ et encore appelée phase lisse (« neat phase »), les rapports de distance sont égaux à : 1, 2, 3, 4, .......Pour la phase paracristalline de type hexagonal directe généralement désignée par H1 ou E et encore appelée phase médian (« middle phase »), les rapports de distance sont égaux à : 1, $\sqrt{3}$, 2, $\sqrt{7}$, ....Aux grands angles de diffraction, les phases paracristallines présentent une bande centrée sur une distance de l'ordre de 4,5 Angströms tandis que les phases cristallines conduisent à des raies fines.

Observations par microscopie optique

Les observations par microscopie optique en lumière polarisée contribuent également à l'identification des phases paracristallines, en particulier lorsque le nombre de raies observées par diffraction des Rayons-X est insuffisant pour établir sans ambiguïté la nature des phases paracristallines en présence.

Les observations de microscopie optique en lumière polarisée sont effectuées par exemple à l'aide d'un microscope LABORLUX S (LEITZ) équipé d'un objectif de grossissement 10, d'un système de polariseurs croisés et d'une platine chauffante (METTLER FP80/FP82). L'échantillon est déposé entre lame et lamelle pour microscope, recouvert par une seconde lame et scellement de l'ensemble par l'intermédiaire d'un joint de Parafilm[R]. Les observations sont effectuées à diverses températures fixes ou en balayage de température à 2°C/min entre la température ambiante et environ 95°C.

Il est connu par exemple que les solutions micellaires isotropes sont non biréfringentes, que les phases paracristallines de type cubique sont également non biréfringentes et que les phases paracristallines de type lamellaire fluide, hexagonal directe ou inverse présentent en lumière polarisée diverses textures caractéristiques décrites par exemple dans « Textures des phases paracristallines rencontrées dans les diagrammes d'équilibre : agents de surface, lipides, eau », F. Lachampt et R.M. Vila, Revue Française des corps gras (1969), 2, 87-111 ou dans « the aqueous phase behavior of surfactants », Robert G. Laughlin Academic press, (1996)-p. 521-546.

## Système tensioactif

Le système tensioactif utilisé dans la composition de l'invention et permettant d'obtenir l'apparition d'une phase paracristalline lors d'un chauffage à au moins 30°C, comprend au moins un tensioactif hydrosoluble et au moins un tensioactif insoluble dans l'eau.

On entend par « tensioactif hydrosoluble » un tensioactif qui à une concentration de 20 g/l dans l'eau permutée à une température d'environ 25°C, donne une solution isotrope transparente.

On entend à l'inverse par « tensioactif insoluble dans l'eau » un tensioactif qui à une concentration de 20 g/l dans l'eau permutée à une température d'environ 25°C, donne une solution trouble indiquant la non solubilisation du tensioactif dans l'eau.

Le ou les tensioactifs insolubles dans l'eau forment une phase dispersée dans le milieu aqueux, cette phase dispersée comprenant tous les composés insolubles dans l'eau.

Le fait d'avoir des tensioactifs insolubles permet d'améliorer les qualités de la mousse obtenue et l'onctuosité de la composition.

## Tensioactifs hydrosolubles

Il s'agit de préférence de tensioactifs moussants, c'est-à-dire aptes à mousser en présence d'eau. Ce sont principalement des dérivés anioniques, non ioniques ou amphotères possédant des chaînes grasses suffisamment courtes pour que ces produits soient bien solubles à température ambiante dans le milieu solvant aqueux de la composition. On peut utiliser un tensioactif hydrosoluble ou un mélange de tels tensioactifs.

Comme tensioactifs hydrosolubles, on cite :

## 1. les tensioactifs anioniques

Selon un mode particulier de réalisation de l'invention, le système tensioactif utilisé comprend de préférence au moins un tensioactif anionique hydrosoluble, et plus particulièrement au moins un acide carboxylique ou un sel d'acide carboxylique hydrosoluble, sel qui est obtenu à partir de l'acide et d'une base. Les acides carboxyliques pouvant être utilisés sont des acides gras, comportant une chaîne alkyle linéaire ou ramifiée, saturée ou insaturée, ayant de 6 à 16 atomes de carbone et de préférence 10 à 14 atomes de carbone. Les sels de tels acides gras constituent des savons. Le fait qu'un savon soit hydrosoluble ou non dépend à la fois de la longueur de la chaîne alkyle et du contre-ion constituant le sel. Comme sels, on peut utiliser par exemple les sels alcalins, les sels alcalino-terreux, les sels d'ammonium, les sels d'aminoalcools et les sels d'aminoacides, et notamment les sels de sodium, de potassium, de magnésium, de triéthanolamine, de N-méthylglucamine, de lysine et d'arginine. Les bases susceptibles d'être utilisées pour obtenir ces sels peuvent être par exemple les bases minérales comme les hydroxydes de métaux alcalins (hydroxyde de sodium et potasse), les hydroxydes de métaux alcalino-terreux (de magnésium) ou l'hydroxyde d'ammonium ou encore les bases organiques comme la triéthanolamine, la N-méthylglucamine, la lysine et l'arginine. L'acide carboxylique peut être en particulier l'acide laurique ou l'acide myristique.

Comme savon hydrosoluble, on peut citer par exemple les sels de potassium des acides gras en C10 à C14, et notamment le sel de potassium de l'acide laurique, le sel de potassium de l'acide myristique et leurs mélanges.

Le savon est généralement introduit dans la composition sous forme de la base d'une part et de l'acide gras d'autre part, la formation du sel se faisant in situ. Ainsi, quand le savon hydrosoluble est constitué du sel de potassium de l'acide laurique et/ou du sel de potassium de l'acide myristique, la composition peut contenir alors de l'acide laurique et/ou de l'acide myristique avec une quantité suffisante de potasse pour former les sels de potassium de l'acide laurique et/ou de l'acide myristique.

Comme autres tensioactifs anioniques pouvant être utilisés dans la composition de l'invention comme tensioactif hydrosoluble, on peut citer par exemple les acides carboxyliques éthoxylés et leurs sels ; les sarcosinates et acylsarcosinates et leurs sels tels que le lauroyl sarcosinate de sodium ; les taurates et méthyltaurates et leurs sels ; les iséthionates et acyl iséthionates, produits de réaction d'acides gras comportant de 10 à 22 atomes de carbone, avec l'acide isethionique, et leurs sels tels que l'iséthionate de sodium et le cocoyl-iséthionate de sodium ; les sulfosuccinates et leurs sels ; les alkylsulfates et alkyléthersulfates et leurs sels, notamment le laurylsulfate de sodium ou de triéthanolamine, et le lauryléther sulfate de sodium ou de potassium ; les monoalkyl- et dialkylesters d'acide phosphorique et leurs sels, tels que par exemple le mono- et dilauryl phosphate de sodium, le mono- et dilauryl phosphate de potassium, le mono- et dilauryl phosphate de triéthanolamine, le mono- et dimyristyl phosphate de sodium, le mono- et dimyristyl phosphate de potassium, le mono- et dimyristyl phosphate de diéthanolamine, le mono- et dimyristyl phosphate de triéthanolamine ; les alkane sulfonates et leurs sels ; les sels biliaires tels que les cholates, déoxycholates, taurocholates, taurodéoxycholates ; les lipoaminoacides et leurs sels tels que les acylglutamates mono- et di-sodiques ; les tensioactifs géminés, bipolaires, tels que décrits dans Surfactant Science series, vol.74 édité par Krister Homberg.

## 2. Tensioactifs amphotères et zwitterioniques

Comme tensioactifs amphotères ou zwitterioniques susceptibles d'être utilisés comme tensioactifs hydrosolubles, on cite les bétaïnes tels que la diméthylbétaïne, la coco-bétaïne et la coco-amidopropylbétaïne ; les sulfobétaïnes tels que la coco-amidopropylhydroxysultaïne ; les alkylamphoacétates tels que cocoamphodiacétate ; et leurs mélanges.

## 3. Tensioactifs non ioniques

Comme tensioactifs non ioniques susceptibles d'être utilisés comme tensioactifs hydrosolubles, on cite les éthers de polyols, comportant des chaînes grasses (8 à 30 atomes de carbone), tels que les éthers gras de sorbitol ou de glycéryle oxyéthylénés ; les éthers et esters de polyglycérol ; les alcools gras polyoxyéthylénés qui sont des éthers formés d'unités d'oxyde d'éthylène et d'au moins une chaîne d'alcool gras ayant de 10 à 22 atomes de carbone, dont la solubilité dépend du nombre d'oxyde d'éthylène et de la longueur de la chaîne grasse ; par exemple, pour une chaîne grasse comportant 12 atomes de carbone, le nombre d'oxyde d'éthylène doit être supérieur à 7, et à titre d'exemple d'alcools gras polyoxyéthylénés, on peut citer les éthers d'alcool laurylique comportant plus de 7 groupes oxyéthylénés ; les alkylpolyglucosides dont le groupe alkyle comporte de 1 à 14 atomes de carbone (alkyl-$C_1$-$C_{14}$ polyglucosides), comme par exemple le décylglucoside, le laurylglucoside, le cocoyl-glucoside ; les alkyl glucopyranosides et alkylthioglucopyranosides ; les alkyl maltosides ; les alcoyl N-méthylglucamides ; les esters de sorbitan polyoxyéthylénés qui comportent généralement de 1 à 100 unités d'éthylène glycol et de préférence de 2 à 40 unités d'oxyde d'éthylène (OE) ; les esters d'aminoalcools ; et leurs mélanges.

Le système tensioactif utilisé dans la composition de l'invention comprend une teneur en tensioactif(s) hydrosoluble(s) allant de 15 à 35 % en poids par rapport au poids total de la composition. Selon un mode préféré de réalisation de l'invention, le système tensioactif dans la composition de l'invention comprend au moins 20 % en poids de tensioactif(s) hydrosoluble(s) par rapport au poids total de la composition.

## Tensioactifs insolubles dans l'eau

Les tensioactifs insolubles dans l'eau apportent notamment la texture (consistance) de la composition finale. Par ailleurs, dans la plage de température comprise entre environ 25°C et 45°C, ces tensioactifs s'associent en partie avec les tensioactifs hydrosolubles pour contribuer à la formation de la phase paracristalline (hexagonale directe) qui est à l'origine de la stabilité du produit jusqu'à au moins 45°C.

Comme tensioactifs insolubles dans l'eau utilisés dans la composition selon l'invention, on cite les acides carboxyliques et leurs sels insolubles dans l'eau, sels obtenus à partir de l'acide et d'une base, et donc les savons insolubles dans l'eau, c'est-à-dire les sels d'acides carboxyliques, comportant une chaîne alkyle linéaire ou ramifiée, saturée ou insaturée, ayant de 6 à 30 atomes de carbone et de préférence 12 à 22 atomes de carbone. Pour les dérivés comportant une seule chaîne grasse saturée, la chaîne comprend avantageusement de 12 à 32 atomes de carbone, de préférence de 14 à 22 atomes de carbone et mieux de 16 à 20 atomes de carbone. Pour les dérivés comportant une chaîne grasse monoinsaturée ou polyinsaturée ou ramifiée, la chaîne comprend avantageusement de 16 à 34 atomes de carbone et de préférence de 18 à 24 atomes de carbone.

Comme acide carboxylique, on peut citer notamment l'acide palmitique et l'acide stéarique.

Comme sels, on peut utiliser les sels alcalins, les sels alcalino-terreux, les sels d'ammonium, les sels d'aminoalcools et les sels d'aminoacides, et notamment les sels de sodium, de potassium, de magnésium, de triéthanolamine, de N-méthylglucamine, de lysine et d'arginine. Les bases susceptibles d'être utilisées pour obtenir ces sels peuvent être par exemple les bases minérales comme les hydroxydes de métaux alcalins (hydroxyde de sodium et potasse), les hydroxydes de métaux alcalino-terreux (de magnésium) ou l'hydroxyde d'ammonium ou encore les bases organiques comme la triéthanolamine, la N-méthylglucamine, la lysine et l'arginine.

On peut citer par exemple comme savon insoluble, le sel de sodium des acides gras en C12 à C22 et le sel de potassium des acides gras en C16 à C22, et notamment le sel de potassium de l'acide palmitique et le sel de potassium de l'acide stéarique.

Le savon est généralement introduit dans la composition sous forme de la base d'une part et de l'acide gras d'autre part, la formation du sel se faisant in situ. Ainsi, quand le savon insoluble est constitué du sel de potassium de l'acide palmitique et/ou du sel de potassium de l'acide stéarique, la composition peut contenir alors de l'acide palmitique et/ou de l'acide stéarique avec une quantité suffisante de potasse pour former les sels de potassium de l'acide palmitique et/ou de l'acide stéarique.

Comme autres tensioactifs pouvant être utilisés dans la composition de l'invention comme tensioactif insoluble, on cite les tensioactifs insolubles non ioniques ou anioniques, comme les esters de glycéryle et d'acides gras comportant de 14 à 30 atomes de carbone, tels que le stéarate de glycéryle ; les alkylpolyglucosides dont le groupe alkyle comporte de 15 à 30 atomes de carbone (alkyl-$C_{15}$-$C_{30}$ polyglucosides), comme par exemple le cétostéarylglucoside ; les dérivés de stérols et de phytostérols éventuellement oxyéthylénés ; les sels alcalins du cholestérol sulfate, et en particulier le sel de sodium ; les sels alcalins du cholestérol phosphate, et en particulier le sel de sodium ; les alcools gras polyoxyéthylénés comportant une chaîne oxyéthylénée ayant un petit nombre de groupes oxyéthylénés, et en particulier moins de 10 groupes oxyéthylénés ; les dialkylphosphates tels que les sels alcalins du dicétylphosphate, et en particulier les sels de sodium et de potassium ; les sels alcalins du dimyristylphosphate, et en particulier les sels de sodium et de potassium ; les lécithines ; les sphingomyélines ; les céramides et leurs mélanges.

Le système tensioactif utilisé dans la composition de l'invention comprend une teneur en tensioactif(s) insoluble(s) dans l'eau allant (en matière active), de 5 à 30 % en poids par rapport au poids total de la composition.

Le système tensioactif (tensioactifs hydrosolubles et insolubles) est présent dans la composition de l'invention en une quantité en matière active, allant de 30 à 65 % en poids et mieux de 40 à 60 % en poids par rapport au poids total de la composition. Le système tensioactif comprend une quantité de savon(s) hydrosoluble(s) d'au moins 10 % en poids par rapport au poids total de la composition, et une quantité totale de savons (hydrosolubles et insolubles) allant de 30 à 40 % en poids par rapport au poids total de la composition.

Le milieu aqueux des crèmes moussantes de l'invention peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition.

Pour obtenir des compositions plus ou moins fluides, on peut incorporer dans les compositions de l'invention un ou plusieurs agents épaississants, notamment des polymères, dans des concentrations préférentielles allant de 0,05 à 2 % en poids par rapport au poids total de la composition.

On peut citer comme exemples d'épaississants :

- les biopolymères polysaccharidiques comme la gomme de xanthane, la gomme de guar, les alginates, les celluloses modifiées ;
- les polymères synthétiques tels que les polyacryliques comme le CARBOPOL 980 commercialisé par la société GOODRICH, les copolymères acrylate/acrylonitrile tels que le HYPAN SS201 commercialisé par la société KINGSTON;
- les épaississants inorganiques tels que les smectites, les hectorites modifiées ou non telles que les produits BENTONE commercialisés par la société RHEOX, les produits LAPONITE commercialisés par la société SOUTHERN CLAY PRODUCTS, le produit VEEGUM HS commercialisé par la société R.T. VANDERBILT ;
- leurs mélanges.

Les compositions selon l'invention constituent des crèmes plus ou moins fluides, dont les modules |G*| ont, à la température de 25°C, des valeurs allant de $10^2$ à $10^5$ Pa et dont les angles de perte $\delta$ vont de 10 à 45° pour des fréquences allant de $10^{-2}$ à 10 Hz.

|G*| et $\delta$ sont les paramètres viscoélatiques utilisés pour mesurer les propriétés physiques des fluides viscoélastiques comme expliqué dans « An introduction to rheology » de H.A. BARNES, J.F. HUTTON, K. WALTERS, pages 46 à 54, (Ed. Elsevier - 1989).

|G*| est le module du module complexe G* (complex modulus) et $\delta$ est l'angle de perte (loss angle). G' et G" sont les composantes de G* : G* = G' + iG". G' et G" sont respectivement le module de conservation (storage modulus) et le module de perte (loss modulus) et i est égal à $(-1)^{1/2}$. Les composantes G' et G" du module complexe sont obtenues à partir de la relation entre la contrainte sinusoïdale (oscillatory stress) et la déformation sinusoïdale (oscillatory strain).

Les mesures rhéologiques de G* et $\delta$ sont effectuées généralement en utilisant un Rhéomètre Haake RS150, à la température de 25°C, avec des corps de mesure de géométrie cône - plan, le diamètre du cône et la dimension du plan étant de 60 mm et l'angle du cône de 2° et l'entrefer entre le cône et le plan étant de 0,1 mm.

Pour faire des mesures dynamiques de viscoélasticité (oscillatory measurements), on détermine d'abord le domaine viscoélastique linéaire, en soumettant l'échantillon à des contraintes sinusoïdales d'amplitudes croissantes et de fréquence constante. Les modules sont reportés en fonction de l'amplitude de la contrainte (amplitude of stress) ou de l'amplitude de la déformation (strain) afin de déterminer les limites du domaine viscoélastique linéaire. Après avoir identifié le domaine viscoélastique linéaire, des mesures dynamiques sont réalisées dans la zone viscoélastique linéaire, pour une valeur de déformation constante située dans le domaine viscoélastique linéaire et à fréquence variable. Le rhéomètre Haake RS150 peut couvrir une gamme de fréquences variant de 0,01 to 10 Hz (soit 0,063 to 62,8 rad/sec).

A partir des valeurs des amplitudes de la contrainte $\tau_0$, de la déformation $\gamma_0$, ainsi qu'à partir du déphasage δ, on établit les relations suivantes :

$$\left| G* \right| = \frac{\tau_0}{\gamma_0}$$

$$G' = \left| G* \right| \cos \delta$$

$$G'' = \left| G* \right| \sin \delta$$

$$G* = G' + iG''$$

Les compositions de l'invention peuvent contenir aussi des adjuvants habituellement utilisés dans le domaine des nettoyants moussants comme les polymères cationiques du type polyquaternium, qui apportent douceur et onctuosité à la crème moussante. Ces polymères cationiques peuvent être choisis de préférence parmi les polymères suivants :

- Polyquaternium 5 tel que le produit MERQUAT 5 commercialisé par la société CALGON ;
- Polyquaternium 6 tel que le produit SALCARE SC 30 commercialisé par la société CIBA, et le produit MERQUAT 100 commercialisé par la société CALGON ;
- Polyquaternium 7 tel que les produits MERQUAT S, MERQUAT 2200 et MERQUAT 550 commercialisés par la société CALGON, et le produit SALCARE SC 10 commercialisé par la société CIBA ;
- Polyquaternium 10 tel que le produit Polymer JR400 commercialisé par la société AMERCHOL ;
- Polyquaternium 11 tel que les produits GAFQUAT 755, GAFQUAT 755N et GAFQUAT 734 commercialisés par la société ISP ;
- Polyquaternium 15 tel que le produit ROHAGIT KF 720 F commercialisé par la société ROHM ;
- Polyquaternium 16 tel que les produits LUVIQUAT FC905, LUVIQUAT FC370, LUVIQUAT HM552 et LUVIQUAT FC550 commercialisés par la société BASF ;
- Polyquaternium 22 tel que le produit MERQUAT 280 commercialisé par la société CALGON ;
- Polyquaternium 28 tel que le produit STYLEZE CC10 commercialisé par la société ISP;
- Polyquaternium 39 tel que le produit MERQUAT PLUS 3330 commercialisé par la société CALGON ;

- Polyquaternium 44 tel que le produit LUVIQUAT CARE commercialisé par la société BASF;
- Polyquaternium 46 tel que le produit LUVIQUAT HOLD commercialisé par la société BASF;
- Polyquaternium 47 tel que le produit MERQUAT 2001 commercialisé par la société CALGON.

On peut aussi utiliser comme polymère cationique les guars cationiques telles que le produit JAGUAR commercialisé par la société RHODIA.

En outre, les compositions de l'invention peuvent contenir des adjuvants habituellement utilisés dans le domaine cosmétique, choisis parmi les huiles, les actifs, les parfums, les conservateurs, les séquestrants (EDTA), les pigments, les nacres, les charges minérales ou organiques telles que le talc, le kaolin, les poudres de silice ou de polyéthylène, les colorants solubles, les filtres solaires. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme exemple d'huile, on peut citer les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthylhexyle, alkyl benzoates), les huiles de silicone volatiles ou non volatiles telles que les polydiméthylsiloxanes (PDMS) et les cyclodiméthylsiloxanes ou cyclométhicones, et les huiles fluorées ou fluorosiliconées, ainsi que les mélanges de ces huiles. La quantité d'huiles ne doit pas modifier la propriété recherchée pour la composition de l'invention : elle est d'au maximum 15 % en poids et mieux d'au maximum 10 % en poids par rapport au poids total de la composition, et elle va de préférence de 0,1 à 5 % en poids et mieux de 0,1 à 3 % en poids par rapport au poids total de la composition.

Comme actifs, on peut citer par exemple les hydratants et, par exemple les hydrolysats de protéines et les polyols tels que la glycérine, les glycols comme les polyéthylène glycols, et les dérivés de sucre ; les extraits naturels ; les oligomères procyannidoliques ; les vitamines ; l'urée ; la caféine ; les dépigmentants tels que l'acide kojique et l'acide caféique ; les alpha-hydroxyacides tels que l'acide lactique et l'acide glycolique ; les rétinoïdes ; les filtres solaires ; les extraits d'algues, de champignons, de végétaux, de levures, de bactéries ; les protéines hydrolysées, partiellement hydrolysées ou non hydrolysées, les enzymes, le coenzyme Q10 ou ubiquinone, les hormones, les vitamines et leurs dérivés, les flavonoides et isoflavones, et leurs mélanges.

Les compositions selon l'invention peuvent constituer notamment des crèmes moussantes pour application topique, utilisées en particulier dans les domaines cosmétique ou dermatologique, comme produits de nettoyage ou de démaquillage de la peau (corps ou visage y compris yeux), du cuir chevelu et/ou des cheveux. Une composition à usage topique contient un milieu physiologiquement acceptable, c'est-à-dire compatible avec la peau, les muqueuses, le cuir chevelu, les yeux et/ou les cheveux. Elle peut constituer plus particulièrement une composition de nettoyage de la peau.

Un autre objet de l'invention consiste en l'utilisation cosmétique de la composition telle que définie ci-dessus, comme produits de nettoyage et/ou de démaquillage de la peau, du cuir chevelu et/ou des cheveux.

Un autre objet de l'invention consiste en un procédé cosmétique de nettoyage des résidus de salissure de la peau, du cuir chevelu et/ou des cheveux, caractérisé par le fait qu'on applique la composition de l'invention, sur la peau, sur le cuir chevelu et/ou sur les cheveux, en présence d'eau, qu'on masse pour former une mousse et qu'on élimine la mousse formée et les résidus de salissure par rinçage à l'eau.

Les exemples qui suivent servent à illustrer l'invention sans toutefois présenter un caractère limitatif. Les quantités indiquées sont en % en poids sauf mention contraire.

Exemple selon l'invention : crème moussante de nettoyage de la peau

- Conservateur          0,4 %
- Tétrasodium EDTA          0,2 %
- Hydroxyde de potassium          7 %
- glycérine          7 %
- PEG-8          7 %
- Acide laurique          3 %
- Acide myristique          20 %
- Acide palmitique          3 %
- Acide stéarique          3 %
- Stéarate de glycéryle (nom CTFA : Glycéryl stéarate SE)          5 %
- cocoyl-glucoside (à 50 % en matière active) (soit 1 % de matière active)          2 %
- Eau          qsp 100 %

Mode opératoire : La phase aqueuse constituée des ingrédients hydrosolubles (eau, conservateurs, EDTA, glycérine, PEG8) est portée à 80°C. La phase grasse constituée des acides gras et du glycéryl stéarate est chauffée et ajoutée sous agitation à la phase aqueuse. Le cocoyl glucoside est ensuite additionné puis la potasse solubilisée dans une partie de l'eau. L'agitation est maintenue 10 minutes à 80°C, puis l'ensemble est refroidi sous agitation.

La composition moussante obtenue est onctueuse et douce.

Les tensioactifs hydrosolubles constitués des sels de potassium des acides laurique et myristique, et du cocoyl-glucoside représentent 29,5 % de la composition, tandis que les tensioactifs insolubles dans l'eau, constitués des sels de potassium des acides palmitique et stéarique et du glyceryl stéarate représentent 12,5 % de la composition. La composition contient ainsi au total 42 % de tensioactifs dont 36 % de savons (KOH + acides laurique, myristique, palmitique et stéarique). Les savons hydrosolubles représentent 28,5 % du poids de la composition.

La composition obtenue a l'aspect d'une crème blanche à température ambiante ; elle se transforme en gel translucide extrêmement visqueux entre 35 et 40°C ; ce gel existe jusqu'à 75-80°C, température à laquelle la composition se fluidifie. Revenu à température ambiante de 25°C, ce gel a de nouveau l'aspect d'une crème homogène.

La stabilité de cette crème est parfaite à 4°C, à la température ambiante et à 45°C pendant au moins deux mois.

Pour cette crème, les valeurs de $|G^*|$ sont de 2900 Pa à 0,01 Hz et de 25000 Pa à 1 Hz, et les valeurs de δ sont de 45° à 0,01 Hz et de 40° à 1 Hz.

Caractérisation:

- A 25°C, la crème est constituée d'une phase micellaire qui a pu être isolée par centrifugation (pendant une heure à 64 000 g, soit à 30 000 tours/minute, avec une centrifugeuse 3K30 Sigma équipée d'un rotor 1210) et d'une phase cristalline. La phase micellaire est transparente, fluide et non biréfringente en lumière polarisée, en diffraction des rayons-X. Cette phase conduit à une raie large aux petits angles centrée autour d'une distance d = 49,8 A et à une bande aux grands angles centrée autour d'une distance d= 4,64 A.

Sur la base des mesures réalisées sur la crème complète, la phase cristalline présente une température de fusion : Tm = 42°C en DSC et est caractérisée en diffraction des rayons-X par 3 raies fines aux petits angles correspondant à des distances d = 42,7 ; 21,4 et 14,2 A et 7 raies fines aux grands angles correspondant à d = 4,37 ; 4,27 ; 4,19 ; 3,92 ; 3,68 ; 3,35 et 3,07 A.

- A 35°C, la crème est homogène à l'échelle macroscopique et constituée du mélange d'une phase hexagonale caractérisée en diffraction des rayons-X par 2 raies fines aux petits angles correspondant à des distances d = 48,7 et 24,3 A et une phase cristalline caractérisée par 3 raies fines aux petits angles correspondant à d = 42,0 ; 21,0 et 14,0 A et 3 raies fines aux grands angles correspondant à d = 4,29 ; 3,92 et 3,09 A.

- A 45°C, la crème est homogène à l'échelle macroscopique et constituée d'un mélange contenant une phase hexagonale caractérisée en diffraction des rayons-X par 2 raies fines aux petits angles correspondant à des distances d = 49,6 et 28,7 A et une phase lamellaire fluide caractérisée par 1 raie fine aux petits angles correspondant à d = 45,0 A. Aux grands angles on observe une bande centrée autour de 4,60 A en accord avec la présence de phases para-cristallines.

- A 55°C, la crème est homogène à l'échelle macroscopique et constituée d'un mélange contenant une phase hexagonale caractérisée en diffraction des rayons-X par 2 raies fines aux petits angles correspondant à des distances d = 47,7 et 27,5 A et d'une phase lamellaire fluide caractérisée par 1 raie faible fine aux petits angles correspondant à d = 36,5 A. Aux grands angles on observe une bande centrée autour de 4,70 A en accord avec la présence de phases paracristallines.

Exemple comparatif :

- Conservateur          0,4 %
- Tétrasodium EDTA        0,2 %
- Hydroxyde de potassium        4 %
- Glycérine       7 %
- PEG-8       7 %
- Acide laurique        3 %
- Acide myristique        3 %
- Acide palmitique        8,7 %
- Acide stéarique        8,7 %
- Stéarate de glycéryle (nom CTFA : Glycéryl stéarate SE      0,75 %
- Lauroyl sarcosinate de sodium (à 30 % en matière active)
  (soit environ 7 % de matière active)        21,8 %
- Eau        qsp 100 %

Le mode opératoire est le même que dans l'exemple 1.
Les tensioactifs hydrosolubles constitués des sels de potassium des acides laurique et myristique, et du lauroyl sarcosinate de sodium représentent 14 % de la composition, tandis que les tensioactifs insolubles dans l'eau, constitués des sels de potassium des acides palmitique et stéarique et du glyceryl stéarate représentent 21,15 % de la composition. La composition contient donc 35,15 % de tensioactifs dont 27,4 % de savons (KOH + acides laurique, myristique, palmitique et stéarique). Les savons hydrosolubles représentent 7 % en poids de la composition.
La composition obtenue a l'aspect d'une crème blanche à température ambiante. Cette composition est bien stable à 4°C, mais elle est instable à 45°C où elle se sépare en deux phases. Revenue à la température ambiante, elle est hétérogène.

- A 25°C, la crème est constituée d'une phase micellaire qui a pu être isolée par centrifugation (pendant une heure à 64 000 g, soit à 30 000 tours/minute avec une centrifugeuse 3K30 Sigma équipée d'un rotor 1210) et d'une phase cristalline.

La phase micellaire est transparente, fluide et non biréfringente en lumière polarisée. En diffraction des rayons-X cette phase conduit à une raie large aux petits angles centrée autour d'une distance d = 50,0 A et à une bande aux grands angles centrée autour d'une distance d= 4,53 A.
Sur la base des mesures réalisées sur la crème complète, la phase cristalline présente une température de fusion : Tm = 45°C en DSC et est caractérisée en diffraction des rayons-X par 5 raies fines aux petits angles correspondant à des distances d = 49,0; 24,1; 16,0; 12,2 et 9,64 A et 6 raies fines aux grands angles correspondant à d = 4,37; 4,28; 4,20; 3,94; 3,66 et 3,08 A.

- A 50°C, il se produit une démixtion macroscopique de la crème en deux phases : Une phase supérieure de type lamellaire fluide qui présente en lumière polarisée une texture caractéristique de type : « Croix de Malte » et conduit en diffraction des rayons-X à une raie fine aux petits angles correspondant à 43,3 A et à une bande aux grands angles centrée autour de 4,78 A.

Une phase inférieure de type solution micellaire, qui est fluide, transparente et non biréfringente en lumière polarisée et caractérisée en diffraction des rayons-X par une raie diffuse aux petits angles correspondant à d = 58,0 A et une bande aux grands angles centrée autour de 4,80 A.
La différence essentielle entre la composition de l'exemple selon l'invention et la composition de l'exemple comparatif porte sur l'aspect macroscopique au-delà de 45°C : la composition selon l'invention donne un système homogène alors que la composition de l'exemple comparatif aboutit à une démixtion.
Pour la composition selon l'invention de l'exemple 1, le système est constitué au-delà de 45°C, d'une phase lamellaire en mélange avec une phase hexagonale directe dont la forte viscosité permet d'éviter la démixtion macroscopique.
Pour la composition de l'exemple comparatif, le système est constitué au-delà de 45°C, d'une phase lamellaire en mélange avec une phase micellaire dont la faible viscosité ne permet pas d'éviter la démixtion macroscopique, aboutissant à une composition hétérogène après retour à la température ambiante.

**Revendications**

1. Composition moussante constituant une crème pour application topique, contenant dans un milieu aqueux, un système tensioactif tel qu'au moins une phase paracristalline de type hexagonale directe apparaisse quand la température augmente au-delà de 30°C et que cette phase paracristalline reste présente jusqu'à au moins 45°C, le système tensioactif étant présent en une quantité de 30 à 65% en poids par rapport au poids total de la composition et comprenant au moins un tensioactif hydrosoluble et au moins un tensioactif insoluble dans l'eau, la quantité de tensioactif(s) hydrosoluble(s) allant de 15 à 35 % en poids par rapport au poids total de la composition, la quantité de tensioactif(s) insoluble(s) allant de 5 à 30 % en poids par rapport au poids total de la composition, la quantité totale de savons allant de 30 à 40 % en poids par rapport au poids total de la composition, dont au moins 10 % en poids de savon(s) hydrosoluble(s) par rapport au poids total de la composition les tensioactifs hydrosolubles étant choisis parmi les acides carboxyliques et leurs sels, les acides carboxyliques éthoxylés et leurs sels, les sarcosinates et acylsarcosinates et leurs sels, les taurates et méthyltaurates et leurs sels, les iséthionates et acyl iséthionates et sels, les sulfosuccinates et leurs sels, les alkylsulfates et alkyléthersulfates et leurs sels, les monoalkyl- et dialkylesters d'acide phosphorique et leurs sels, les alkane sulfonates et leurs sels, les sels biliaires, les lipoaminoacides et leurs sels, les tensioactifs géminés et leurs mélanges; les bétaïnes, les sulfobétaïnes, les alkylamphoacétates est leurs mélanges; les éthers de polyols, les éthers et esters de polyglycérol, les alcools gras polyoxyéthylénés, les alkyl-$C_1$-$C_{14}$ polyglucosides, les alkyl glucopyranosides et alkylthioglucopyranosides, les alkyl maltosides, les alcoyl N-méthylglucamides, les esters de sorbitan polyoxyéthylénés, les esters d'aminoalcools et leurs mélanges; et
les tensioactifs insolubles étant choisis parmi les acides carboxyliques et leurs sels ; les esters de glycéryle et d'acides gras ; les alkyl-$C_{15}$-$C_{30}$ polyglucosides ; les dérivés de stérols et de phytostérols éventuellement oxyéthylénés ; les sels alcalins du cholestérol sulfate ; les sels alcalins du cholestérol phosphate ; les alcools gras polyoxyéthylénés ; les dialkylphosphates ; les lécithines ; les sphingomyélines ; les céramides et leurs mélanges.

**2.** Composition selon la revendication 1, **caractérisée en ce qu'**elle a un module |G*| allant, à la température de 25°C, de $10^2$ à $10^5$ Pa et un angle de perte δ allant de 10 à 45° pour des fréquences allant de $10^{-2}$ à 10 Hz.

**3.** Composition selon l'une quelconque des revendications précédentes, **caractérisée en ce qu'**elle comprend au moins un solvant choisi parmi les alcools inférieurs ; les polyols ; les sucres et leurs mélanges.

**4.** Composition selon l'une quelconque des revendications précédentes, **caractérisée en ce qu'**elle comprend au moins un agent épaississant.

**5.** Composition selon l'une quelconque des revendications précédentes, **caractérisée en ce qu'**elle constitue une composition de nettoyage de la peau.

**6.** Utilisation cosmétique d'une composition selon l'une quelconque des revendications 1 à 4, comme produits de nettoyage et/ou de démaquillage de la peau, du cuir chevelu et/ou des cheveux.

**7.** Procédé cosmétique de nettoyage des résidus de salissure de la peau, du cuir chevelu et/ou des cheveux, **caractérisé en ce qu'**on applique la composition selon l'une quelconque des revendications 1 à 4, sur la peau, sur le cuir chevelu et/ou sur les cheveux, en présence d'eau, qu'on masse pour former une mousse et qu'on élimine la mousse formée et les résidus de salissure par rinçage à l'eau.

**Claims**

**1.** Foaming composition constituting a cream for topical application comprising, in an aqueous medium, a surface-active system such that at least one paracrystalline phase of direct hexagonal type appears when the temperature increases above 30°C and that this paracrystalline phase remains present up to at least 45°C, the surface-active system being present in an amount of 30 to 65% by weight, with respect to the total weight of the composition, and comprising at least one water-soluble surfactant and at least one water-insoluble surfactant, the amount of water-soluble surfactant(s) ranging from 15 to 35% by weight, with respect to the total weight of the composition, the amount of insoluble surfactant(s) ranging from 5 to 30% by weight, with respect to the total weight of the composition, the total amount of soaps ranging from 30 to 40% by weight, with respect to the total weight of the composition, including at least 10% by weight of water-soluble soap(s), with respect to the total weight of the composition, the water-soluble surfactants being chosen from:

carboxylic acids and their salts, ethoxylated carboxylic acids and their salts, sarcosinates and acyl sarcosinates and their salts, taurates and methyl taurates and their salts, isethionates and acyl isethionates and their salts, sulphosuccinates and their salts, alkyl sulphates and alkyl ether sulphates and their salts, monoalkyl and dialkyl esters of phosphoric acid and their salts, alkanesulphonates and their salts, bile salts, lipoamino acids and their salts, geminal surfactants and their mixtures;

betaines, sulphobetaines, alkylamphoacetates and their mixtures;

polyol ethers, polyglycerol ethers and esters, polyoxyethylenated fatty alcohols, ($C_1$-$C_{14}$)alkyl polyglucosides, alkyl glucopyranosides and alkylthio glucopyranosides, alkyl maltosides, alkyl-N-methylglucamides, polyoxyethylenated sorbitan esters, amino alcohol esters and their mixtures; and

the insoluble surfactants being chosen from:

carboxylic acids and their salts; esters of glycerol and of fatty acids; ($C_{15}$-$C_{30}$) alkyl polyglucosides; derivatives of sterols and of phytosterols which are optionally oxyethylenated; alkaline salts of cholesterol sulphate; alkaline salts of cholesterol phosphate; polyoxyethylenated fatty alcohols; dialkyl phosphates; lecithins; sphingomyelins; ceramides and their mixtures.

2. Composition according to Claim 1, **characterized in that** it has a modulus |G*| ranging, at a temperature of 25°C, from $10^2$ to $10^5$ Pa and a loss angle δ ranging from 10 to 45° for frequencies ranging from $10^{-2}$ to 10 Hz.

3. Composition according to either one of the preceding claims, **characterized in that** it comprises at least one solvent chosen from lower alcohols; polyols; sugars and their mixtures.

4. Composition according to any one of the preceding claims, **characterized in that** it comprises at least one thickening agent.

5. Composition according to any one of the preceding claims, **characterized in that** it constitutes a composition for cleaning the skin.

6. Cosmetic use of a composition according to any one of Claims 1 to 4, as products for cleaning and/or removing makeup from the skin, scalp and/or hair.

7. Cosmetic method for cleaning dirt residues from the skin, scalp and/or hair, **characterized in that** the composition according to any one of Claims 1 to 4 is applied to the skin, scalp and/or hair in the presence of water, **in that** massaging is carried out in order to form a foam and **in that** the foam formed and the dirt residues are removed by rinsing with water.

## Patentansprüche

1. Schaumbildende Zusammensetzung für die topische Anwendung, bei der es sich um eine Creme handelt und die in einem wässrigen Medium ein Tensidsystem enthält, das so ist, dass mindestens eine parakristalline Phase vom Typ hexagonal direkt auftritt, wenn die Temperatur über 30 °C steigt, und diese parakristalline Phase bis mindestens 45 °C erhalten bleibt, wobei das Tensidsystem in einer Menge von 30 bis 65 Gew.-%, bezogen auf das Gesamtgewicht der Zusammensetzung, enthalten, ist und mindestens einen wasserlöslichen grenzflächenaktiven Stoff und mindestens einen in Wasser unlöslichen grenzflächenaktiven Stoff enthält, wobei der Mengenanteil des wasserlöslichen grenzflächenaktiven Stoffes oder der wasserlöslichen grenzflächenaktiven Stoffe im Bereich von 15 bis 35 Gew.-%, bezogen auf das Gesamtgewicht der Zusammensetzung, liegt, der Mengenanteil des unlöslichen grenzflächenaktiven Stoffes oder der unlöslichen grenzflächenaktiven Stoffe im Bereich von 5 bis 30 Gew.-%, bezogen auf das Gesamtgewicht der Zusammensetzung, liegt, die Gesamtmenge der Seifen im Bereich von 30 bis 40 Gew.-%, bezogen auf das Gesamtgewicht der Zusammensetzung, liegt, davon mindestens 10 Gew.-% wasserlösliche Seife(n), bezogen auf das Gesamtgewicht der Zusammensetzung, wobei die wasserlöslichen grenzflächenaktiven Stoffe unter Carbonsäuren und ihren Salzen, ethoxylierten Carbonsäuren und ihren Salzen, Sarcosinaten und Acylsarcosinaten und ihren Salzen, Tauraten und Methyltauraten und ihren Salzen, Isethionaten und Acylisethionaten und ihren Salzen, Sulfosuccinaten und ihren Salzen, Alkylsulfaten und Alkylethersulfaten und ihren Salzen, Monoalkyl- und Dialkylestern von Phosphorsäure und ihren Salzen, Alkansulfonaten und ihren Salzen, biliären Salzen, Lipoaminosäuren und ihren Salzen, Gemini-Tensiden und deren Gemischen; Betainen, Sulfobetainen, Alkylamphoacetaten und deren Gemischen; Polyolethern, Ethern und Estern von Polyglycerin, polyethoxylierten Fettalkoholen, $C_{1-14}$-Alkylpolyglucosiden, Alkylglucopyranosiden und Alkylthioglucopyranosiden, Alkyl.maltosiden, Alkyl-N-methylglucamiden, ethoxylierten Sorbitanestern, Estern von Aminoalkoholen und deren Gemischen; und die unlöslichen grenzflächenaktiven Stoffe unter Carbonsäuren und ihren Salzen; Glycerylfettsäureestern; $C_{15-30}$-Alkylpolyglucosiden; Derivaten von Sterolen und Phytosterolen, die gegebenenfalls ethoxyliert sind; Alkalisalzen von Cholesterinsulfat; Alkalisalzen von Cholesterinphosphat; polyethoxylierten Fettalkoholen; Dialkylphosphaten; Lecithinen; Sphingomyelinen; Ceramiden und deren Gemischen ausgewählt sind.

2. Zusammensetzung nach Anspruch 1, **dadurch gekennzeichnet, dass** sie bei einer Temperatur von 25 °C einen Modul |G*| von $10^2$ bis $10^5$ Pa und für Frequenzen von $10^{-2}$ bis 10 Hz einen Verlustwinkel δ von 10 bis 45° aufweist.

3.  Zusammensetzung nach einem der vorhergehenden Ansprüche, **dadurch gekennzeichnet, dass** sie mindestens ein Lösungsmittel enthält, das unter den niederen Alkoholen; Polyolen; Zuckern und deren Gemischen ausgewählt ist.

4.  Zusammensetzung nach einem der vorhergehenden Ansprüche, **dadurch gekennzeichnet, dass** sie mindestens ein Verdickungsmittel enthält.

5.  Zusammensetzung nach einem der vorhergehenden Ansprüche, **dadurch gekennzeichnet, dass** es sich um eine Zusammensetzung zur Reinigung der Haut handelt.

6.  Kosmetische Verwendung einer Zusammensetzung nach einem der Ansprüche 1 bis 4 als Produkt für die Reinigung und/oder zum Abschminken der Haut, der Kopfhaut und/oder der Haare.

7.  Kosmetisches Reinigungsverfahren zum Entfernen von Schmutzrückständen von der Haut, der Kopfhaut und/oder den Haaren, **dadurch gekennzeichnet, dass** die Zusammensetzung nach einem der Ansprüche 1 bis 4 auf die Haut, die Kopfhaut und/oder die Haare in Gegenwart von Wasser aufgebracht und zur Bildung eines Schaums massiert wird und der gebildete Schaum und die Schmutzrückstände durch Spülen mit Wasser entfernt werden.